Europäisches Patentamt

European Patent Office

Office européen des brevets

(19)

(11) **EP 1 591 535 A1**

(12) **EUROPEAN PATENT APPLICATION**

(43) Date of publication:
02.11.2005 Bulletin 2005/44

(51) Int Cl.7: **C12Q 1/68**

(21) Application number: **04076309.6**

(22) Date of filing: **29.04.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK**<br><br>(71) Applicant: **Nederlandse Organisatie voor Toegepast-Natuurwetenschappelijk Onderzoek TNO**<br>**2628 VK Delft (NL)**<br><br>(72) Inventors:<br> • **Schuren, Frank Henri Johan**<br>  **3902 EB Veenendaal (NL)** | • **Caspers, Martinus Petrus Maria**<br>  **2289 EG Rijswijk (NL)**<br> • **van der Werff-van der Vat, Bianca Johanna C.**<br>  **6717 SP Ede (NL)**<br> • **Montijn, Roy Christiaan**<br>  **1024 NL Amsterdam (NL)**<br><br>(74) Representative:<br>**Winckels, Johannes Hubertus F. et al**<br>**Vereenigde**<br>**Johan de Wittlaan 7**<br>**2517 JR Den Haag (NL)** |

(54) **Classification of organisms based on genome representing arrays**

(57)    The invention relates to nucleic acid arrays and methods for analysing arrays and typing sample nucleic acid tested on the array. In one aspect the invention provides a method for typing sample nucleic acid comprising, providing an array comprising a plurality of nucleic acid molecules wherein said array nucleic acid molecules comprise an average size of between about 200 to 5000 nucleotides and hybridizing said array with sample nucleic acid, wherein said sample nucleic acid comprises an average size of between about 50 to 5000 nucleotides and comparing the sample hybridization pattern with a reference pattern. In the invention it is not necessary to have detailed knowledge of the sequences that are present in the array.

EP 1 591 535 A1

**Description**

The invention relates to the fields of array technology, diagnostics and molecular biology.

Array technology has become an important tool in various fields related to biology and medicine. Several types of arrays have been developed through the years. With the advent of miniaturization and automation more and more information has been entered into arrays. The current trend in array technology is to generate ever-larger arrays, carrying more and more information on them.

The present invention follows this trend; arrays of the invention contain at least 500.000 nucleotides on them. Preferably the arrays carry even more nucleotides on them. In a preferred embodiment the array comprises at least 1 megabase ($10^6$ nucleotides). Preferably, they comprise at least 2 megabases. Contrary to conventional arrays, the number of bases per spot is high, i.e. between 200 and 5000 nucleotides. In conventional arrays, the number of nucleotides per spot is kept low for reasons of economy and precision. Generating larger nucleotides increases the price of the oligonucleotide and simultaneously introduces a larger propensity for errors with respect to the desired sequence. In the present invention, this drawback is, at least in part, overcome though the preferred use of nucleic acid that is obtained or derived from a natural source, preferably living material. The introduction of larger numbers of nucleotides in the array also introduces another difficulty, particularly when complex nucleic acid is used to probe the array. In this situation a larger number of spots have signals between the value 0 and 1, indicative for the fact that not all of the nucleic acid in the spot is hybridized to probe nucleic acid. Although arrays of the invention can be used to identify specific sequences in the probe nucleic acid, and thus to type the probe nucleic acid on the basis of a signal in one or a few spots, the full potential of the array lies in the interpretation of all the signals obtained in the spots. This interpretation can be done by a human but is typically done by a computer using statistical software.

Thus in one aspect the invention provides a method for typing sample nucleic acid comprising, providing an array comprising a plurality of nucleic acid molecules wherein said array nucleic acid molecules comprise an average size of between about 200 to 5000 nucleotides and hybridizing said array with sample nucleic acid comparing the sample hybridization pattern with a reference pattern. It is not necessary to have detailed knowledge of the sequences that are present on the array. In the present invention patterns are compared with each other. The average size of sample nucleic acid has an effect on the signal distribution on the array. Larger sample molecules comprise more information and are thus more likely to find a suitable hybridization partner in more of the spots. Reducing the average size of the sample nucleic acid can reduce this phenomenon. On the other hand, when the sample nucleic acid is too small the nucleic acid fragments in the sample contain too little genetic information and also find suitable hybridization partners in many spots. The average size of the fragments in the sample nucleic acid is preferably between about 50 and 5000 nucleotides. More preferably, the average size of the fragments in the sample nucleic acid comprises a size of between about 50 and 1000 nucleotides, more preferably between about 50 and 500 nucleotides.

The sample nucleic acid preferably represents the whole sample genome. The pattern obtained with the sample nucleic acid on the array is compared with a reference pattern. The reference pattern can be artificially generated for instance through using knowledge of the nucleic acid composition of a reference sample, for instance the genome sequence of an organism of which the genomic sequence is known. However, in a preferred embodiment, the reference pattern is generated by hybridizing reference nucleic acid to the array. Comparison of the sample pattern with the reference can at least be used to determine whether the sample nucleic acid is the same or similar to the reference nucleic acid. This is useful when one needs to determine whether, for instance, the sample nucleic acid contains a particular prokaryote. In this setting a reference pattern is generated with nucleic acid of the particular prokaryote and when the sample pattern is essentially the same as the reference pattern, the sample is identified as containing the particular prokaryote. In a preferred embodiment a method of the invention further comprises comparing the sample pattern with at least one other reference pattern. In this way the sample can be compared to at least two different reference nucleic acids. Of course, upon continued use of the array, more and more patterns are generated and all of these can be used to compare with the sample nucleic acid. Thus once a pattern is generated with sample nucleic acid this pattern can in a subsequent experiment be used as a reference pattern. Thus in a preferred embodiment a method of the invention further comprises comparing the hybridization pattern with at least 2 and preferably at least 5 reference patterns. More preferably at least 10, more preferably at least 100 reference patterns. The sample pattern and the reference pattern can be a subset of signals obtained from the array. A pattern can consist of one signal; preferably, the pattern consists of at least 20 % of the signals obtained following hybridization to the array. More preferably, the pattern consists of at least 50% of the signals from the array. In a particularly preferred embodiment the pattern comprises at least 80% of the signals of the array.

A method of the invention cannot only be used to determine whether a sample nucleic acid is the same as a particular reference nucleic acid. Particularly, when prokaryotic nucleic acid is used as a sample nucleic acid, the sample pattern can, as it happens, be different from any of the reference patterns. A particularly useful characteristic of the methods and arrays of the invention is that also in this case a method of the invention can provide useful information. Phenotypic characteristics associated with the organism from which the sample nucleic acid is derived or obtained are very often the result of the interplay of a large number of different sequences and/or genes. In these situations it is not possible to type a particular sample on the basis of the signal obtained in one or more spots. Rather, the signals of very many different spots need to be compared. The methods and arrays of the invention are particularly suited for this type of analysis. To this end the reference patterns and the sample pattern are analyzed using statistical software. In one embodiment, a method of the invention further comprises unsupervised multivariate analysis (PCA) of the reference patterns together with the pattern generated by the sample nucleic acid. Based on this analysis a pattern is given an n dimensional value (with n representing the total number of datapoints included in the analysis) which can be reduced to its principal components, preferably two, these components can be visualized in a multidimensional visualization, preferably in a two-dimensional visualization. The dimensional value of the components can be plotted for all patterns which are included in the analysis whereupon the grouping or clustering of the preferably two-dimensional values of the patterns can be scrutinized. In a preferred embodiment the two-dimensional value of the sample pattern is compared with all two-dimensional values of the reference patterns. In this way it is possible to provide a statistical estimation of the relatedness of the organism that the sample nucleic acid is obtained or derived from compared to the included references

In another embodiment of the invention patterns are extended with further information on the organism wherefrom a reference and/or sample nucleic acid is obtained or derived. For instance, patterns may be extended with parameters that are determined in a way different from nucleic acid hybridization. For instance, when prokaryotes are the objects of study, it is often important to know the antibiotic resistance phenotype of the prokaryote. This resistance parameter can be added to the statistical analysis. The value of this parameter (resistant or not resistant, or further fine tuning) can be added to the pattern or to the statistical analysis of the patterns. The clustering can subsequently be based on this additional parameter. Thus in a preferred embodiment of the invention, at least one non nucleic acid based parameter is determined for the organisms that were the source of the reference patterns. The statistical analysis can subsequently be used to determine or estimate the value of this parameter for the organism that was the source of the sample nucleic acid. In this embodiment, a method of the invention further comprises Partial Least Square-Discriminant Analysis (PLS-DA) of the reference patterns together with the pattern generated by the sample nucleic acid, wherein at least one parameter of which the values are known for the reference patterns is used to supervise the PLS-DA analysis.

**Partial Least Squares (PLS)**

Partial least squares (PLS) has been described extensively in the literature (P. Geladi and B.R. Kowalski, Partial Least Squares Regression: A Tutorial, *Analytica Chimica Acta,* 185, 1986, 1-17. H. Martens and T. Naes, Multivariate Calibration, John Wiley & Sons, Chichester, 1989.) Whereas a principal component analysis (PCA) model has a *descriptive* nature, a PLS model has a *predictive* nature. In PLS, scores*loadings pairs, also called latent variables (LVs), are not calculated to maximise the explained variance in the *predicting data set* only, but also to maximise the covariance with the *data to be predicted.* The PLS model can be summarised mathematically by means of Equation (1) and Equation (2).

$$X = TP^T + E \tag{1}$$

$$Y = TBQ^T + F \tag{2}$$

Matrix **X** (also called X-block) represents a $n * p$ matrix of independent variables ($n$ chromatograms, for example, with $p$ retention times per chromatogram), **Y** (also called Y-block) is a $n * q$ matrix containing the dependent variables (concentrations, for example); **P**$^T$ and **Q**$^T$ are transpose $S * p$ and $S * q$ matrices, containing the dependent and independent variable loadings, respectively; **T** is an $n * S$ matrix of $S$ latent scores, **B** is a $S * S$ matrix representing the regression of the scores of the **X** matrix on the scores of the **Y**-data; **E** and **F** are $n * p$ and $n * q$ matrices containing the residuals of the independent and dependent variables, respectively.
The standard error of validation (SEV) after extracting A LVs, is calculated from Equation (3).

$$SEV = \sqrt{\frac{\sum_{l=1}^{I_c} \left(Y_{i,j} - y_{i,j}\right)^2}{I_c}} \tag{3}$$

where $I_c$ is the number of calibration samples, $y_{i,j}$ is the true value for the concentration of component *j* in object i; $Y_{i,j}$ is the PLS predicted value for $y_{i,j}$; *q* is the number of *Y*-variables. The extraction of LVs is continued as long as the SEV is improved significantly.

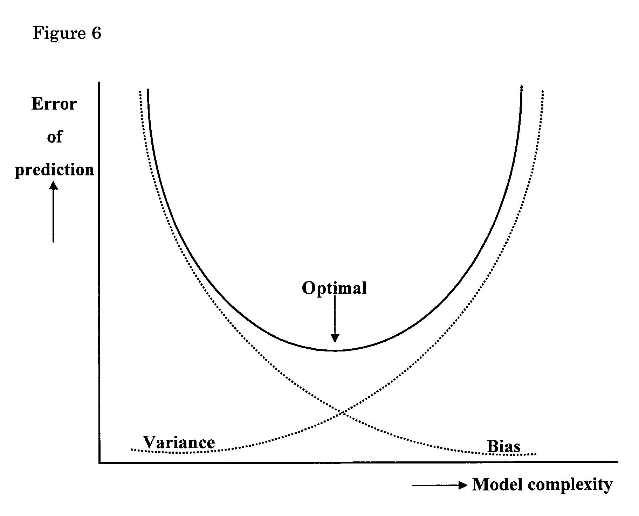

The number of LVs chosen must yield an optimal prediction of the variable of interest. However, there is a pay-off between variance and bias (or fit): a too complex model fits well, but may predict very poorly. This leads to the concept of optimal model complexity: an optimal balance between fit and variance is obtained. This is illustrated graphically in Figure 6, where the increased complexity of the model is able to fit more features in the data, but the variance of the estimated parameters rises and the overall result attains a minimum value at the optimal model complexity.

Pure linear relations between **X** and **Y** will result in a simple model with usually two to five LVs. Complex non-linear relations can also be modelled. However, these will take up significantly more LVs to correlate **Y** to **X**.

## Partial Least Squares - Discriminant Analysis (PLS-DA)

In PLS-DA classes (predefined groups) are used as dependent variables. The Y-block **Y** is a matrix of n*number of classes. The Y-block is filled with zeros and ones.

Example:

$$class = [1\ 2\ 2\ 1]$$

$$Y = \begin{bmatrix} 1 & 0 \\ 0 & 1 \\ 0 & 1 \\ 1 & 0 \end{bmatrix}$$

Using a Y-block in PLS which is filled with zeros and ones dependent on the class each sample belongs to, turns PLS into a discriminant analysis.

## Principal Component-Discriminant Analysis (PC-DA)

Discriminant analysis (DA) [D.L. Massart, B.G.M. Vandeginste, L.M.C. Buydens, S. De Jong, P.J. Lewi and J. Smeyers-Verbeke, Handbook of Chemometrics and Qualimetrics: Part A, Elsevier, Amsterdam, 1997; B.G.M. Vandeginste, D.L. Massart, L.M.C. Buydens, S. De Jong, P.J. Lewi and J. Smeyers-Verbeke, Handbook of Chemometrics and Qualimetrics: Part B, Elsevier, Amsterdam, 1998] is applied if the interest is focused on differences between groups of samples. The technique is based on the assumption that samples of the same group are more similar compared to samples of other groups. The goal of DA is to find and identify structures in the original data, which show large differences in the group means. This process involves a priori knowledge of which samples are similar. Therefore, DA is said to be a *supervised* analysis technique. This distinguishes it from other *unsupervised* techniques such as principal component analysis (PCA), for example, which does not require a priori knowledge about samples.

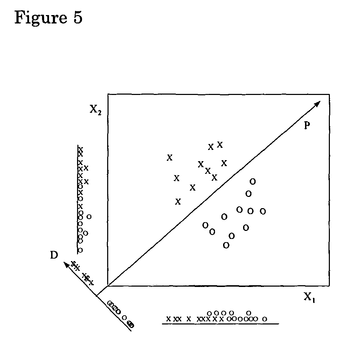

The first step in DA is to combine the original variables into a set of mutually independent new variables in such a way that the projection of the original samples in the space, spanned by a minimum number of these new variables, maximises the difference between the group means. This principle is demonstrated in Figure 5. Two groups of samples are measured on two variables $X_1$ and $X_2$. Using the principal component (PC) maximum variance criterion these samples should be projected on the line through the samples as indicated in Figure 5 by line P. For discriminating between the different clusters of samples this is not the optimal solution. However, the projection of the samples on line D shows a complete separation between the two clusters. The calculated factors are called discriminants or D-axes. All other projections give sub-optimal solutions. This is demonstrated in the figure 5 by comparing the projection of the samples on de D-line with those on the $X_1$ or $X_2$ axis.

DA describes most efficiently the differences between groups of samples. However, the number of variables is often large compared to the number of samples. This may lead to degenerate solutions. For instance three samples can always be separated by two variables independent of their similarity. If more samples are included this degeneracy effect will disappear. The general rule of thumb is that the number of samples should be at least four times the number of variables. This rule can lead to problems in the examination of nuclear magnetic resonance (NMR) spectra, for example. In the analysis of natural products the number of peaks (variables) per NMR spectrum is generally in the order of a few hundred. Under normal circumstances this would mean that one should measure at least 400 to 800 samples. In practice this never occurs. Based on this it would be impossible to perform DA on NMR spectra of natural products. However, there is a solution to this problem. Hoogerbrugge *et al.* [R. Hoogerbrugge, S.J. Willig and P.G. Kistemaker, Discriminant Analysis by Double Stage Principal Component Analysis, *Analytical Chemistry,* 55, 1983, 1710-1712.] developed a scheme in which the number of variables is reduced by PCA, firstly, followed by DA on the scores of the samples on the first PC axes. This technique is called principal component-discriminant analysis (PC-DA). Determining the exact number of PCs to include is difficult. The number should not be too small because including only the first few can result in a loss of a lot of the between-group information. The number should not be too large also, because it will exceed the number-of-samples-divided-by-four rule. Therefore, it seems advisable to include all PCs, which explain a significant amount of variance (for instance above 1% of the original variance) up to a maximum of the number of samples divided by four. If the total amount of variance explained by these PCs is very low then the number can always be increased. However, if the explained variance is low, the correlations between the original variables will be low also. As a consequence DA will generate a result which will be as complicated as the original problem.

The parameter used in the PLS-DA analysis is preferably a phenotypic parameter. The term "phenotypic parameter" is here used to define any parameter that describes any property that is exhibited or expressed by the organism or a functional part of the organism. Based on this analysis a pattern is given an n-dimensional value (with n representing the total number of discriminating datapoints included in the analysis) which can be reduced to its, preferably two, principal components for optimal correlation with the phenotypic character in a, preferably, two-dimensional visualization. This preferably two-dimensional value can be plotted for all patterns which are included in the analysis whereupon the grouping or clustering of the preferably two-dimensional values of the patterns can be scrutinized. In a preferred embodiment the two-dimensional value of the sample pattern is compared with all two-dimensional values of the reference patterns. In this way it is possible to provide a statistical estimation of the chance that the organism that the sample nucleic acid is obtained or derived from comprises a certain phenotypic characteristic or not. This of course necessitates that this phenotypic characteristic is known for the organisms from which the reference nucleic acids are obtained or derived from. In a preferred embodiment the two-dimensional values of the reference patterns are clustered based on the supervised PLS-DA analysis. This clustering is preferably done on the basis of the phenotypic characteristic for which the sample pattern is scrutinized. This clustering preferably results in two clusters, wherein one cluster has the certain phenotype and the other has not. The sample pattern can thus easily be identified as having or not having the certain phenotype. A method of the invention preferably further comprises typing said sample nucleic acid on the basis of the presence or absence in a cluster. This typing is typically associated with a statistical margin of error for the classification, i.e. the statistical chance that the sample nucleic acid is wrongly classified as having or not having the certain phenotypic characteristic. The borders of the clusters can be set to accommodate a smaller or larger statistical chance of error. In a preferred embodiment the parameter comprises antibiotic resistance, epidemic character, pathogenicity, virulence, commensalism, heat resistance, pH tolerance, persistence, cell death and other characteristics of potential interest.

A similar approach can be followed for a wide variety of nucleic acids. As mentioned above, the array is preferably generated from a nucleic acid obtained or derived from a natural source. This source can be of viral, microbial, animal or plant origin. In the case of eukaryotic origin it is preferred that the nucleic acid from the organism is first put through some type of selection system such that repetitive nucleic acid is at least in part removed prior to generating the array. In this way it is prevented that the array contains a lot of redundant information. One way to achieve this is to select sequences that encodes a functional RNA in the eukaryote. This so-called coding nucleic acid typically comprises little if any repetitive nucleic acid. Alternatively, selections are based on other methods. One such other method to enrich for unique sequences is to hybridize nucleic acid from an eukaryotic organism under conditions wherein repetitive nucleic acid is preferably hybridized (making use of Cot curves). Hybridized nucleic acid can be separated from single stranded nucleic acid whereupon the single stranded nucleic acid can be amplified and/or cloned. In a preferred embodiment the source is a simple eukaryote, preferably a single cell eukaryote. These sources contain simpler genomes and accordingly less redundant nucleic acid. In a particularly preferred embodiment, the source is a prokaryote. A prokaryote hardly contains redundant nucleic acid and thus no specific selection step need to be performed for generating an efficient array. In a special embodiment of a prokaryote, a eukaryotic cell organelle containing nucleic acid that is thought to be derived from a prokaryotic ancestor is used as a source of nucleic acid for array construction and/or sample nucleic acid.

In a particularly preferred aspect of the invention nucleic acid obtained or derived from at least one prokaryotic strain is used to generate a reference pattern. Preferably, at least 5 and more preferably at least 50 reference patterns are generated by nucleic acid obtained or derived from different prokaryotic strains. In a particularly preferred embodiment essentially all reference patterns are generated by nucleic acid of prokaryotic strains. Preferably, the different prokaryotic strains belong to the same prokaryotic genus. In this way it is possible to type a sample for the presence therein of nucleic acid derived from a certain prokaryotic genus. More preferably the different prokaryotic strains belong to the same prokaryotic species. In this way it is possible to type a sample for the presence therein of nucleic acid derived from a certain strain of a particular prokaryotic species. This particularly preferred embodiment is preferably combined with a statistical analysis of the reference and sample pattern. In this way it is possible to determine the chance that a prokaryote in a sample comprises a certain phenotypic characteristic or genotypic relatedness of some but not all strains of a certain prokaryotic species. The prokaryotic nucleic acid can be derived from RNA but is preferably derived or obtained from prokaryotic DNA. i.e. derived from the genome. Thus in a preferred embodiment of the invention nucleic acid molecules are derived from prokaryotic DNA.

Sample nucleic acid preferably comprises nucleic acid derived from the same organism, genus, species or strain as the organism, genus, species or strain used to generate the reference patterns. The sample and/or reference nucleic acid used to generate the patterns may contain a subset of nucleic acid of the organism, genus, species or strain it is derived or obtained from. However, preferably, no selections, other than those mentioned for eukaryotic sources are performed. In any case, preferably selections are the same or similar for sample and reference nucleic acid. This allows for an easy comparison of the reference and the sample patterns.

With the term nucleic acid obtained or derived from it is meant that it is not essential that the nucleic acid used to hybridize on the array is directly obtained from the source. It may have undergone cloning, selections and other manipulations prior to the use for hybridizations. Sample and reference nucleic acid can for instance be obtained from cloned libraries, such as expression or genome libraries. Alternatively, sample and reference nucleic acid can be generated from scratch based on the nucleic acid information in databases, for instance, as a result of the ongoing genomics efforts. However, preferably sample and reference nucleic acid are obtained directly, or through amplification from a natural source. A sample may contain a mixture of organisms, for instance, in the case of a sample obtained from flora containing a plurality of microorganisms. In this case, reference patterns generated from various microbiological floras can be used to compare the sample patterns against or with. As mentioned above, a natural source is preferably a prokaryotic source. Preferably, the sample and reference patterns are generated starting from a monoculture of a prokaryote. In this way it is warranted that only one organism is analyzed on the array, and in the same time the pattern generated is a pattern generated from a prokaryotic strain.

In one aspect, the invention provides an array comprising a plurality of nucleic acid molecules wherein said nucleic acid molecules comprise an average size of between about 200 to 5000 nucleotides. Preferably, the nucleic acid molecules comprise an average size of between about 200 and 5000 nucleotides. An array of the invention preferably comprises at least 500.000 nucleotides on them. Preferably the arrays carry even more nucleotides on them. In a preferred embodiment the array comprises at least 1 megabase ($10^6$ nucleotides). Preferably, they comprise at least 2 megabases. Contrary to conventional arrays, the number of bases per spot is high, i.e. more than 200 nucleotides, preferably the number of bases is between 200 and 5000 nucleotides. Preferably, said plurality of nucleic acid molecules are derived from a natural source. Preferably, wherein said plurality of nucleic acid molecules is derived from prokaryotic DNA. It has been found that different strains of a prokaryote, while belonging to the same species can nevertheless vary greatly in the kind of DNA that they carry. Thus in a preferred embodiment, an array of the invention comprises a plurality of nucleic acid molecules that is derived from at least two different strains of prokaryotes, preferably of the same species. In this way the array is a more representative of the entire genetic diversity of a prokaryotic species. In a particularly preferred embodiment, the array comprises a plurality of nucleic acid molecules that is derived from at least three different strains of prokaryotes, preferably of the same species. By increasing the number of strains of a prokaryotic species to generate the plurality of nucleic acids in the array, the array more and more mimics the complete genetic potential of a prokaryotic species and thus the typing becomes more and more informative. This does not mean that typing with arrays carrying a reduced number of different prokaryotic strains is not a valid approach; it only means that predictions and estimations become more accurate and complete.

Examples

**Example 1**

**Classification of *Staphylococcus aureus* species by (unsupervised) PCA- analysis of whole-genome-array differential hybridisation data.**

Fluorescently labeled genomic DNA's (gDNA) of a set of 31 different *Staphylococcus aureus* species were separately hybridised to arrays coated with randomly chosen gDNA fragments of a mixture of 8 different S. *aureus* species (approx. 2100 fragments/array, approx. 1500bp/fragment). The fluorescent hybridisation patterns were quantified resulting in a list of hybridisations signals per genomic DNA fragment for each tested species. To be more specific each array was simultaneously hybridised with 2 labeled gDNA's: one concerning a specific S. *aureus* species under investigation (labeled with Cy5), and the other concerning a standard mix of the 8 *S. aureus* species used for making of the array serving as a reference to normalise hybridisations made on all the separate slides (labeled with Cy3). The subsequent data analysis involved filtering, normalisation and cut-off-treatment of the data followed by Principal Component Analysis (PCA). This resulted in clustering of similar S. *aureus* species based on whole genome differential hybridisations. Reproducibility was shown by duplicate hybridisations for some species.

*Set of different bacterial strains*

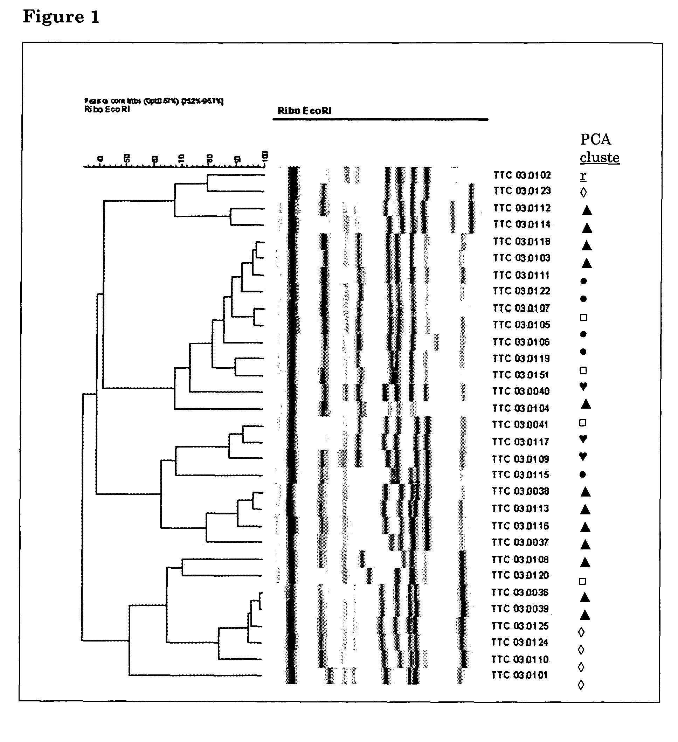

A set of 31 S. *aureus* strains was used for Example 1 (Fig.1). The set consisted of 30 hospital isolates and 1 reference strain from a type-strain collection (Fig.1, strain TTC.03.151). Riboprint analysis of part of their ribosomal DNA (DuPont Qualicon, 3531 Silverside Rd, Bedford Building, Wilmington, DE 19810) indicated various degrees of relation between the different strains of the set (Figure 1).

*Growth and gDNA isolation of Staphylococcus strains*

*Staphylococcus* isolates were grown (via single colonies) on TSA-agar plates and/or TSA-medium (overnight, 37°) and stored as glycerol cultures (-80°). For gDNA isolation, plate grown bacteria (e.g. amount of 10-20 kolonies) were resuspended in 400 µl TE-buffer (10 mM Tris, 1 mM EDTA, pH7.5) in a 2 ml vial. The cells were lysed by adding 400 µl water-washed 0.1 mm Zirconium glassbeadsuspension (Biospec Products™), precooling on ice, medium-level shaking for 120 sec in a cell disrupter (minibeadbeater 8, Biospec Products™) and cooling on ice. After centrifugation (5 min, 14 krpm, 4°C), gDNA was isolated from the cleared lysate according to standard procedures (Sambrook, 1989) by extraction with phenol/chloroform/isoamylalcohol (roomtemp.), extraction with chloroform/isoamylalcohol (roomtemp.), precipitation with ethanol/Na-acetate (-20°C, spinning at 4°), washing with 70% ethanol (-20°C, spinning at 4°), drying (vacuum), dissolving the pellet in 100 µl TE-buffer with RNAseA (1-100 µg/ml) and semi-quantification of the gDNA-amount on 0.6% agarose Ethidiumbromide gels (e.g. 1-5 µl preparation/slot).

*Construction of S.aureus gDNA array (slides)*

To make an array containing a genome-wide represention of the species *S.aureus,* a mixed-genomic library of the organism was made by mixing gDNA of 8 *S.aureus* strains. Strains were selected that: (a) showed each a different profile of resistance to a broad set of antibiotics (together covering most types of antibiotic-resistance), and (b) did not contain a significant plasmid band in the agarose-gel analysis of their isolated gDNA. The gDNA mix was sheared by sonication (Branson sonifier 450) and runned in several lanes of a 0.8% agarose gel. DNA-fragments (approx. 1-3 kb) were excised and isolated via binding to glass-milk (Bio101-kit). The isolated fragments were pretreatment with DNA-terminator End-repair kit (Lucigen Corp.) to facilitate efficient (blunt)cloning into bacterial plasmids (pSmartHCkan vector, CloneSmart Blunt Cloning Kit, Lucigen). Part of the ligationmix (1µl) was transformed to 25 µl *E.coli* cells *(E.kloni* 10G supreme electrocompetente cells, Lucigen) by electroporation (0,1cm-gap cuvettes Eurogentec, BioRad Pulsor, 25 uF, 200 ohms, 1,6 kV) and regeneration in TB-culture medium and plating on TY-plates with 30 µg/ml kanamycin grown overnight at 37°C. Using tooth-picks, colonies were transferred to into 96-well microtiterplates (32 plates, 150 µl/well TY medium with 30 µg/ml kanamycin). After overnight growing at 37°C, glycerol was added (final conc. 15%) and the glycerol-stocks were stored at -80°C.
The genomic inserts from each clone in the well-plates were multiplied using PCR-amplification in 96-well PCR-plates (22 plates). PCR reactions contained 50 µl reactionmix/well with 1xSuperTaq buffer, 0.2mM of each dNTP (Roche Diagnostics), 0.4 µM primer L1(5'-cag tcc agt tac gct gga gtc-3') and 0.4 µM primer L1(5'-ctt tct gct atg gag gtc agg tat g-3'), 1.5 U SuperTaq-DNA-polymerase and 1µl glycerolstock from corresponding well of gDNA-bank. Both primers contain a free NH2-group coupled via a C6-linker to the 5' end of the primer. The following PCR-program was used: 4 min 94°C, 30x (30 sec 94°C, 30 sec 50°C, 3 min 72°C), 10 min 72°C and soaking at 4°C. Following the amplifications, the 50 µl PCR-products were transferred to 96-well roundbottom plates and precipitated by adding 150 µl NaAc/isopropanol mix (0.2M NaAc, 67% isopropanol final conc. each), incubation 1hr -80°C, spinning (1 hr, 2.5 krpm, 4°C), removal of supernatant and washing with 100 µl 70% ethanol. DNA-pellets were resuspended in 50 µl water/well, transferred to 384-well plates, dried (speed vac) and resuspended in 10 µl 3xSSC-buffer per well. The 6 resulting 384-well plates, containing approx. 2100 PCR-products were used for spotting the micro-arrays. The PCR-products were spotted on series of maximal 75 "aldehyde" coated slides (Cell Associates)) using an ESI micro-array printer in combination with 24 TeleChem Stealth micro spotting quill-pins (approx 100 µm diameter). After spotting, slide surfaces were blocked by treatment at room temperature with boro-anhydride: 2x 5min in 0.2% SDS, 2x 5min in water, 10 min in boro-anhydride buffer (1.7 g NaBH4 in 510 ml PBS-buffer and 170 ml 100% ethanol), 3x 5min in 0.2% SDS, 3x 5min in water, 2sec in 100°C water, dry with $N_2$ flow. PBS (phosphate buffered saline) is 6.75 mM Na2HPO4, 1.5 mM K2HPO4, 140 mM NaCl, 2.7 mM KCl pH 7.0. (1.2 g Na2HPO4, 0.2 g K2HPO4, 8.0 g NaCl, 0.2 g KCl per liter, pH 7.0).

*Labeling of gDNA*

Fluorescent labeling of gDNA was performed on 0.5-2 µg of isolated Staphylococcus gDNA for 1.5 hr at 37° in a 25 µl reaction based on BioPrime$^R$ DNA Labeling System (Invitrogen, Cat. No.: 18094-011). The reaction contained (final conc): 1x RandomPrimer solution (50mM Tris-HCl PH 6.8, 5mM MgCl2, 30µg/ml random octamers, Bioprime$^R$), 1x lowT dNTP-mixture (0.25mM dATP, 0.25mM dGTP, 0.25mM dCTP, 0.1mM dTTP), 0.06mM Cy-dUTP (Cy = either Cy5 or Cy3, 1µl of 1mM stock, Amersham Biosciences) and 20 Units DNA-polymerase (Klenov fragment; 0.5 µl of 40U/µl stock, Bioprime$^R$). After the reaction, salts, unincorporated (labeled) nucleotides and primers were removed by purification over an Autoseq G50 column (Amersham Biosciences). After purification 1/10$^{th}$ part of the labeled material was used for spectrophotometic analysis to determine quantity of DNA (A260$^{nm}$) and Cy5 (A649$^{nm}$) or Cy5 (A550$^{nm}$). The remainder of the labelled material was used for array hybridisation.

*(Pre-)Hybridisation of arrays*

In preparation for hybridisation, slides were laid in Petri dishes, in 20 ml prehybridisation solution (1%BSA,, 5xSSC, 0.1°/SDS, filtered through a 0.45 µm filter, 42°) and were gently shaken (by mild rotation) for 45 min at 42°.
Next the slide was washed 2x in 40 ml water (in a 40 ml capped tube) and quickly dried using an $N_2$-gun. The appropriate gDNA samples that were labeled with Cy5-dUTP and Cy3-dUTP were combined with 4 µl yeast tRNA (25 µg/µl), dried (using a SpeedVac), re-dissolved in 40 µl EasyHyb solution (Roche Applied Science), denatured (1.5 min, 95°C), spinned down briefly (1 sec, 10 krpm), pipetted on a pre-warmed (42°C metal plate) dry prehybridised array, covered with a plastic cover slip (Hybrislip, Molecular Probes), inserted in a water-vapour-saturated prewarmed (42°C) hybridisation chamber (Corning) and hybridised overnight in a 42°C waterbath. After hybridisation the arrays were washed by shaking slides 4x in 40ml of (different) buffers in capped 40ml tubes (wash-buffer1: 1x SSC, 0.2%SDS, 37°, 5-10 sec; wash-buffer2: 0.5x SSC, 37°C, 5-10 sec; wash-buffer3 and 4: 0.2x SSC, 20°C, each 10 min).

*Scanning and image analysis*

After washing, slides were stored in the dark (to prevent decay of Cy-fluorescence) or directly used for scanning the fluorescent Cy dyes with a scanning device (ScanArray 4000 from Perkin Elmer with Scanalyse software from PackardBioscience). A quickscan (resolution 30 µm/pixel) was performed to select optimal laser- (intensity) and detection (photomultipier) settings in order to prevent excess of low signals or saturated signals. Slides were 2 times scanned: for Cy5- and Cy3-fluorescence. Digital scans were quantified with Imagene software (BioDiscovery Inc., version 4.2) resulting in a spot identity, and Signal (S) and Background (B) values for both Cy5 and Cy3, for each spot on the array. The data were stored in electronic files and used for further data processing.

*Data pre-processing*

By using speadsheet software (Excel, Microsoft) the following calculations were made for each spot: S-B values for Cy3 and Cy5, Cy5/Cy3 ratio's [R=Cy5(S-B)]/[(Cy3(S-B)]. Low quality data were removed (e.g. spots having Cy3 data with S<2B). Then, a normalisation factor N was calculated for each slide, based on average Cy5- and Cy3-signal for all spots on a slide (N = [averageCy5(S-B)]/[averageCy3(S-B)]. Next, normalised ratio's ($R_n$) were calculated for each spot ($R_n$ = R/N) on all arrays. A matrix (= dataset) of normalised ratio's per spot for many slides (slides relate with *Staphylococcus* strains) was used for further data preprocessing.
Since the Cy3 signal is generally present for most spots (Cy3-labeled reference gDNA pool of 8 strains was hybridised to all slides), and the Cy5 signal can vary (Cy5-labeled gDNA of different strains were each hybridised to single slides), the Cy5/Cy3 ratio can in theory have two values (1 or 0) if a gDNA fragment is present or absent respectively, in the Cy5 tested strain. In practice, however, these values vary around 1 and 0. Therefore, in many analyses cut-off values for 0 and 1 were applied on the ratio-dataset before further analysis (e.g. $R_n$<0.5 and $R_n$>0.5 were replaced by 0 and 1 respectively or, $R_n$<0.3 and $R_n$>0.7 were replaced by 0 and 1 respectively while keeping $R_n$ values between 0.3 and 0.5). These "cut-off datasets" were used for the final data analysis.

*PCA data analysis*

A "cut-off dataset" was analysed by Principle Component Analysis (PCA) with Mean- Centring as the selected scaling method. Comparable results can be obtained with no or alternative scaling methods (e.g. autoscaling) or alternative multivariate methods.

*Results*

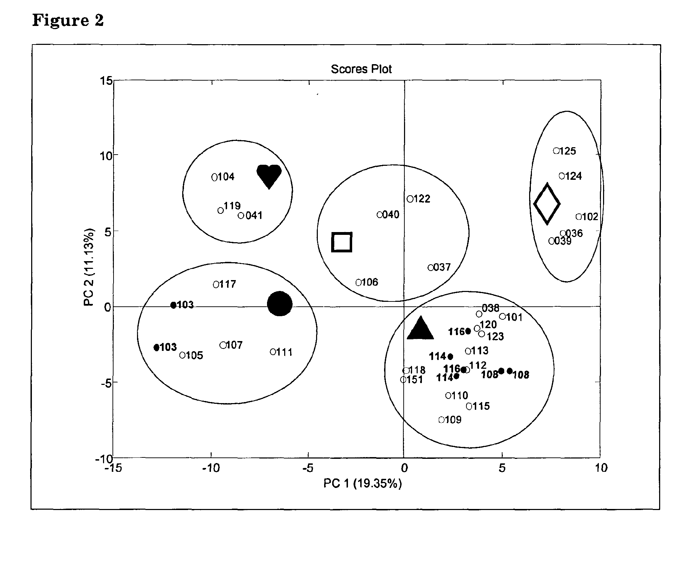

A set of 31 *S.aureus* strains was processed for the established Riboprint classification method (Fig.1) and for the new classification method based on PCA analysis of data from differential hybridisation on whole-genome micro-arrays (Fig.2). Comparison of Figure 1 and 2 shows that the new method (Fig.2) results in a significantly different strain clustering than the established method (Fig.1). Because the array/PCA-method (Fig.2) is based on whole-genome differential hybridisation it has a higher potential for classification of close related organisms than classic classification methods based only on specific DNA sequences (e.g. ribosomal DNA sequence polymorphisms, Fig.1) or based only on limited phenotypic information (e.g. growth conditions, bacteria staining etc.).

**Example 2**

**Phenotypic classification for resistance to antibiotics of *Staphylococcus* species by (supervised) PLS-DA analysis of whole-genome-array differential hybridisation data.**

Fluorescently labeled genomic DNA's (gDNA) of a set of 25-31 different *S.aureus* species with known resistance/sensitivity to 4 different antibiotics were separately hybridised to arrays coated with randomly chosen genomic DNA fragments of a mixture of 8 different *S.aureus* species (approx. 2100 fragments/array, approx. 1500bp/fragment). The fluorescent hybridisation patterns were quantified resulting in a list of hybridisations signals per genomic DNA fragment for each tested species. To be more specific each array was simultaneously hybridised with 2 labeled gDNA's: one concerning a specific *S.aureus* species under investigation (labeled with Cy5), and the other concerning a standard mix of the 8 *S.aureus* species used for making of the array serving as a reference to normalise hybridisations made on all the separate slides (labeled with Cy3).
The subsequent data analysis involved filtering, normalisation and cut-off-treatment of the data followed by Principal Least Square Discrimminant Analysis (PLS-DA) on basis of the known sensitivity/resistance of the strains to 4 antibiotics (1 antibiotic per analysis). The 4 independent PLS-DA analyses of the same dataset (only slightly differing in the amount of analysed strains) resulted in 4 significant clusterings of the *S.aureus* species according to their known sensitivity/resistance against each of the 4 antibiotics. For each antibiotic the sensitive and resistant cluster contained a different subset of strains according to their known antibiotic resistance/sensitivity. This indicates that for each antibiotic, a different part of the total differential hybridisation dataset contains antibiotic specific information.

*Set of different bacterial strains*

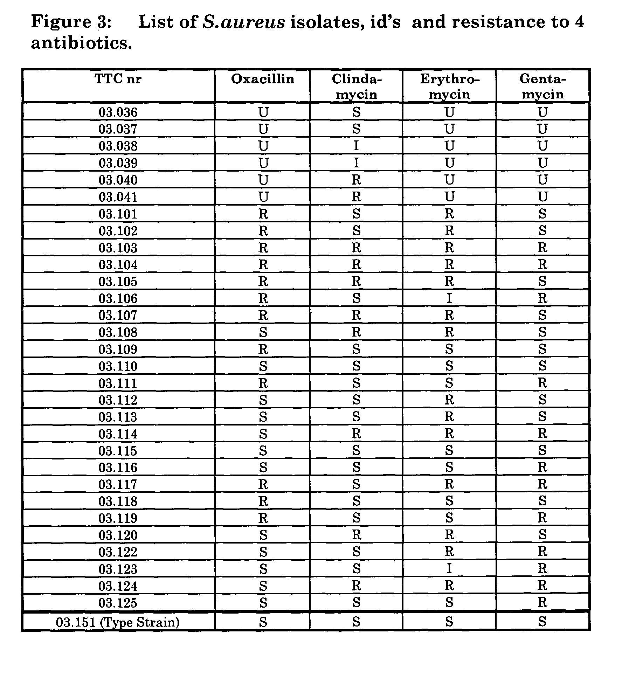

A set of 25-31 *S.aureus* strains was used for Example 2 (Fig.3). The set consisted of 30 hospital isolates and 1 reference strain from a type-strain collection (Fig.3, strain TTC.03.151). For almost all strains their antibiotic resistance/sensitivity was determined by agar-diffusion test for 4 different antibiotics (Clindamycin, Erythromycin, Gentamycin and Oxacillin).

*Growth and gDNA isolation of Staphylococcus strain, Construction of Staphylococcus gDNA array (slides), Labeling of gDNA, (Pre-)Hybridisation of arrays, Scanning and image analysis, Data pre-processing*

The above-mentioned procedures were performed as described in Example 1.

*PLS-DA data analysis*

A "cut-off dataset" was analysed by Principal Least Square Discrimminant Analysis (PLS-DA) on basis of the known sensitivity/resistance of the strains to a single antibiotic (a "supervised" method).
Comparable results can be obtained with no or alternative scaling methods (e.g. autoscaling) or alternative multivariate methods.

*Results*

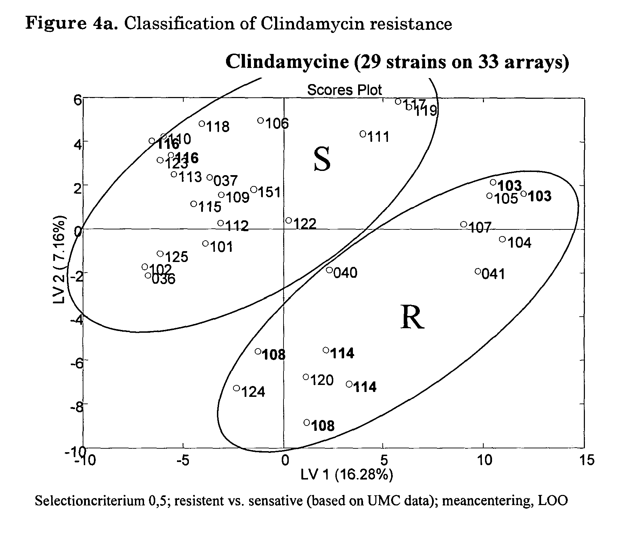

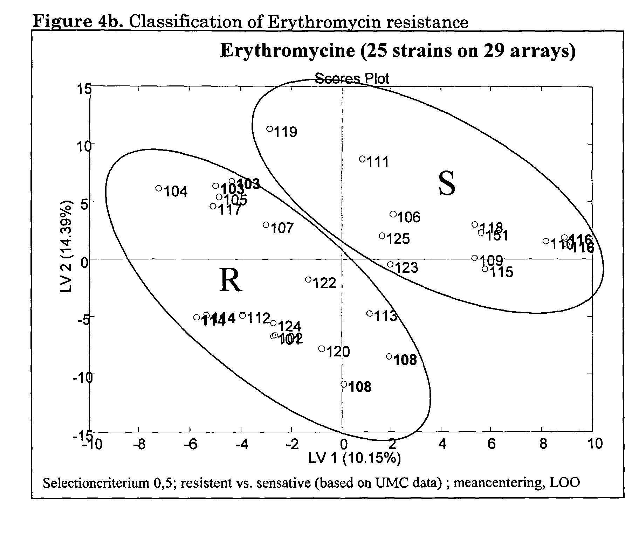

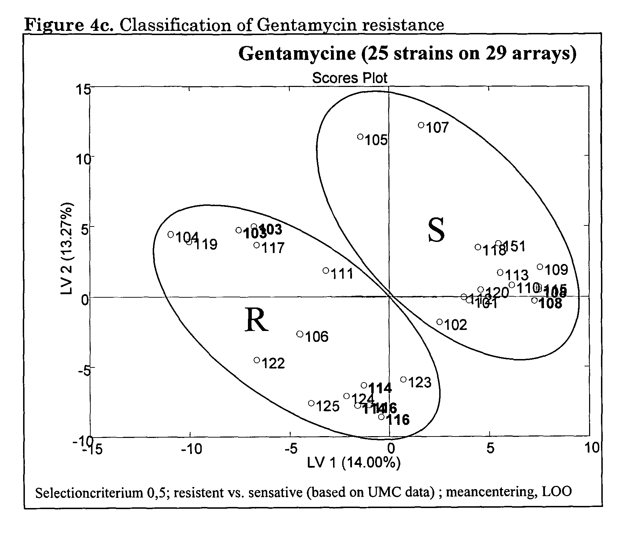

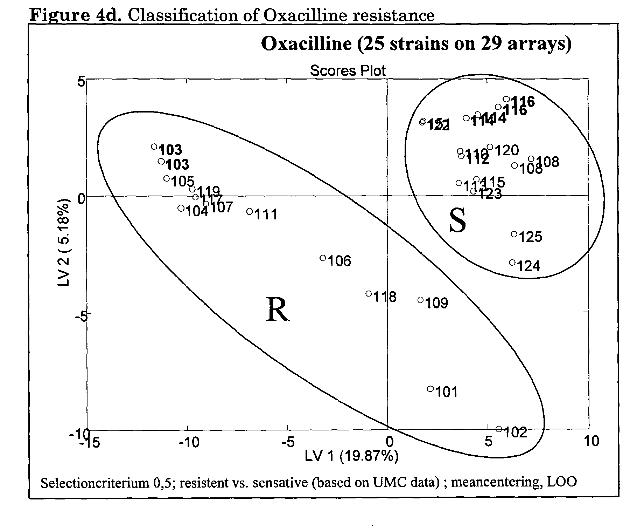

A set of 25-31different *S.aureus* isolates was processed for PLS-DA analysis of data from differential hybridisation on whole-genome microarrays (Fig.4). The 4 independent PLS-DA analyses of the same dataset (only slightly differing in the amount of analysed strains) resulted in 4 significant clusterings of the *S.aureus* species according to their known sensitivity/resistance against each of the 4 antibiotics. For each antibiotic the sensitive and resistant cluster contains a different subset of strains according to their known antibiotic resistance/sensitivity. This shows that for each antibiotic, a different part of the total differential hybridisation dataset contains antibiotic specific information. The antibiotic-specific whole-genome hybridisation data can be used to predict the antibiotic resistance/sensitivity of unknown *S.aureus* strains.

**References**

**[0041]**   - Sambrook, J., E.F. Fritsch and T. Maniatis

**Brief description of the drawings**

**Figure 1**

**Riboprint classification of 31 *Staphylococcus aureus* species used in the study.**

Most strains are *S.aureus* hospital isolates. One reference strain is from a strain-type collection (TTC 03.151). The figure shows the specific riboprint pattern of each strain (banding pattern in the middle) and a dendrogram (left) indicating the degree of (Pearson-)correlation between the riboprint patterns. At the right, for each strain is given the TTC number (TNO Type Collection). Symbols (▲,□, ♥ ,●,⌘) at the far right indicate the strain classification according to the PCA-clusters of Figure 2.

**Figure 2**

**Classification of *Staphylococcus* species by (unsupervised) PCA-analysis of whole-genome-array differential hybridisation data.**

Cy-labelled genomic DNA of 31 different *Staphylococcus aureus* strains was hybridised to 35 arrays (4 strains in duplo) containing a representation of the *Staphylococcus aureus* genome. The quantified fluorescent hybridisation patterns of the *Staphylococcus* species, representing a highly complex n-dimensional data-set, were analysed with Principle Component-Analysis (PCA). The PCA-plots below shows single point projections of each single-species complex hybridisation pattern in a 2-dimensional plane (small circles, with text indicating strain TTC.03 number). Duplo hybridised strains are indicated with filled small circles and bold text. For reasons of clarity, strains projected close together are manually clustered by ellipses. Each cluster was identified by a symbol (▲,□, ♥ , ●, ⌘) also indicated at the right of each Riboprint classified strain in Figure 1. Note: Cy5/Cy3-ratio's were transformed to a 0 and 1 dataset by cutt-off 0.5; PCA-scaling was Meancentering.

**Figure 3: List of *S.aureus* isolates, id's and resistance to 4 antibiotics.**

Resistance to antibiotics was determined by classic agardiffusion-test (columns 2-5: U = Unknown, S = Sensitive, I = intermediate, R = Resisitant). Studied *S.aureus* strains are indicated by TNO type strain nr (TTC nr). All strains are hospital isolates, except the last one is from a type strain collection.

**Figure 4: Phenotypic classification for resistance to antibiotics of *Staphylococcus* species by (supervised) PLS-DA analysis of whole-genome-array differential hybridisation data.**

Cy-labelled genomic DNA of 31 different *Staphylococcus* strains was hybridised to arrays containing a representation of the *Staphylococcus* genome. The quantified fluorescent hybridisation patterns of the *Staphylococcus* species, representing a highly complex n-dimensional data-set, were analysed with Partial-Least-Square-Discrimminant-Analysis (PLS-DA) on basis of the known antibiotic sensitivity (S) or resistance (R) of each *S.aureus* strain. The PLS-DA plots below show single point projections of each single-species complex hybridisation pattern in a 2-dimensional plane (small circles, with text indicating strain TTC.03 number). Duplo hybridised strains are indicated with bold text. Based on a specific part of the dataset, the PLS-DA analysis is able to cluster the species in two separate clusters (manually placed ellipses, indicated S and R) according to their known antibiotic resistance to 4 different antibiotics **(Fig. 4a-d)**. Note: Cy5/Cy3-ratio's were transformed to a 0 and 1 dataset by cutt-off 0.5; PLS-DA-scaling was Meancentering.

Figure 5. Illustration of discriminant analysis. D is the discriminant axis, P is a projection line, X1 and X2 are two variables and x and o represent samples from two different groups.

Figure 6. Model complexity.

**Claims**

1. A method for typing sample nucleic acid comprising, providing an array comprising a plurality of nucleic acid molecules wherein said array nucleic acid molecules comprise an average size of between about 200 to 5000 nucleotides and hybridizing said array with sample nucleic acid, wherein said sample nucleic acid comprises an average size of between about 50 to 5000 nucleotides and comparing the sample hybridization pattern with a reference pattern.

2. A method according to claim 1, wherein said plurality of nucleic acid molecules is derived from a natural source.

3. A method according to claim 1 or claim 2, wherein said plurality of nucleic acid molecules is derived from prokaryotic DNA.

4. A method according to claim 3, wherein said plurality of nucleic acid molecules is derived from at least two different species of prokaryotes.

5. A method according to claim 3 or claim 4, wherein said plurality of nucleic acid molecules is derived from at least two different prokaryotic strains belong to the same genus.

6. A method according to claim 3, wherein said plurality of nucleic acid molecules is derived from at least two different prokaryotic strains belong to the same species.

7. A method according to any one of claims 1-6, wherein said plurality of nucleic acid molecules is derived from a pure culture of a prokaryote.

8. A method according to claim 1 or claim 2, wherein said plurality of nucleic acid molecules is derived from eukaryotic DNA.

9. A method according to any one of claims 1-8, further comprising comparing the hybridisation pattern with at least one other reference pattern.

10. A method according to claim 9, comprising comparing the hybridization pattern with at least 2, more preferably at least 5 and even more preferably at least 50 reference patterns.

11. A method according to claim 10, wherein said comparison comprises unsupervised multivariate analysis of the reference patterns together with the pattern generated by the sample nucleic acid.

12. A method according to claim 11, further comprising clustering representations of patterns based on Principal Component Analysis (PCA) .

13. A method according to claim 12, further comprising typing said sample nucleic acid on the basis of the presence or absence in a cluster.

14. A method according to claim 10, wherein said comparison comprises Partial Least Square-Discriminant Analysis (PLS-DA) of the reference patterns together with the sample pattern and wherein at least one phenotypic parameter of which the values are known for the reference patterns, (and which information is used to supervise the PLS-DA analysis), is additionally determined or estimated for the sample nucleic acid or the natural source it is derived from.

15. A method according to claim 14, further comprising clustering representations of patterns based on the supervised PLS-DA analysis.

16. A method according to claim 15, further comprising typing said sample nucleic acid on the basis of the presence or absence in a cluster.

17. A method according to any one of claims 14-16, wherein said cluster represents patterns sharing a value for a phenotypic parameter or characteristic of interest.

18. A method according to any one of claims 14-17 wherein said parameter is antibiotic resistance.

**19.** A method according to any one of claims 14-18 wherein said parameter is epidemic character, pathogenicity, virulence, commensalism, heat resistance, pH tolerance, persistence, cell death and others.

**20.** A method according to any one of claims 9-19, wherein at least one of said reference patterns is a pattern generated by nucleic acid from a prokaryotic strain.

**21.** A method according to any one of claims 9-20, wherein essentially all reference patterns are generated by nucleic acid of prokaryotic strains belonging to the same prokaryotic genus.

**22.** A method according to claim 20, wherein essentially all reference patterns are generated by nucleic acid of prokaryotic strains belonging to the same prokaryotic species.

**23.** A method according to any one of claims 9-19, wherein at least one of said reference patterns is a pattern generated by nucleic acid from a eukaryote.

**24.** A method according to claim 23, wherein essentially all reference patterns are generated by nucleic acid of eukaryotes belonging to the same eukaryotic genus.

**25.** A method according to claim 23 or claim 24, wherein essentially all reference patterns are generated by nucleic acid of eukaryotes belonging to the same eukaryotic species.

**26.** An array comprising a plurality of nucleic acid molecules, preferably representing the whole genome of an organism, wherein said nucleic acid molecules comprise an average size of between about 200 to 5000 nucleotides and preferably contains at least 500.000 nucleotides.

**27.** An array according to claim 26, wherein said plurality of nucleic acid molecules are derived from a natural source.

**28.** An array according to claim 27, wherein said plurality of nucleic acid molecules is derived from prokaryotic DNA.

**29.** An array according to claim 28, wherein said plurality of nucleic acid molecules is derived from at least two different strains of prokaryotes.

**30.** An array according to claim 26, wherein said plurality of nucleic acid molecules is derived from eukaryotic DNA

**31.** An array according to claim 30, wherein said plurality of nucleic acid molecules is derived from at least two different eukaryotes belonging to the same eukaryotic genus, preferably belonging to the same species.

**Figure 1**

**Figure 2**

**Figure 3:** List of *S.aureus* isolates, id's and resistance to 4 antibiotics.

| TTC nr | Oxacillin | Clinda-mycin | Erythro-mycin | Genta-mycin |
|---|---|---|---|---|
| 03.036 | U | S | U | U |
| 03.037 | U | S | U | U |
| 03.038 | U | I | U | U |
| 03.039 | U | I | U | U |
| 03.040 | U | R | U | U |
| 03.041 | U | R | U | U |
| 03.101 | R | S | R | S |
| 03.102 | R | S | R | S |
| 03.103 | R | R | R | R |
| 03.104 | R | R | R | R |
| 03.105 | R | R | R | S |
| 03.106 | R | S | I | R |
| 03.107 | R | R | R | S |
| 03.108 | S | R | R | S |
| 03.109 | R | S | S | S |
| 03.110 | S | S | S | S |
| 03.111 | R | S | S | R |
| 03.112 | S | S | R | S |
| 03.113 | S | S | R | S |
| 03.114 | S | R | R | R |
| 03.115 | S | S | S | S |
| 03.116 | S | S | S | R |
| 03.117 | R | S | R | R |
| 03.118 | R | S | S | S |
| 03.119 | R | S | S | R |
| 03.120 | S | R | R | S |
| 03.122 | S | S | R | R |
| 03.123 | S | S | I | R |
| 03.124 | S | R | R | R |
| 03.125 | S | S | S | R |
| 03.151 (Type Strain) | S | S | S | S |

**Figure 4a.** Classification of Clindamycin resistance

**Clindamycine (29 strains on 33 arrays)**

Selectioncriterium 0,5; resistent vs. sensative (based on UMC data); meancentering, LOO

**Figure 4b.** Classification of Erythromycin resistance

Erythromycine (25 strains on 29 arrays)

Selectioncriterium 0,5; resistent vs. sensative (based on UMC data) ; meancentering, LOO

EP 1 591 535 A1

**Figure 4c.** Classification of Gentamycin resistance

**Gentamycine (25 strains on 29 arrays)**

Scores Plot

Selectioncriterium 0,5; resistent vs. sensative (based on UMC data) ; meancentering, LOO

**Figure 4d.** Classification of Oxacilline resistance

Oxacilline (25 strains on 29 arrays)

Scores Plot

Selectioncriterium 0,5; resistent vs. sensative (based on UMC data) ; meancentering, LOO

Figure 5

Figure 6

**EP 1 591 535 A1**

**European Patent Office**

# EUROPEAN SEARCH REPORT

Application Number

EP 04 07 6309

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/187490 A1 (CHO JAE-CHANG ET AL) 12 December 2002 (2002-12-12) * abstract * * paragraph [0003] * * paragraph [0007] - paragraph [0011] * * claims 1-21 * ----- | 1-31 | C12Q1/68 |
| X | CHO J-C ET AL: "Bacterial species determination from DNA-DNA hybridization by using genome fragments and DNA microarrays" APPLIED AND ENVIRONMENTAL MICROBIOLOGY, WASHINGTON,DC, US, vol. 67, no. 8, August 2001 (2001-08), pages 3677-3682, XP002953482 ISSN: 0099-2240 * abstract * * page 3677, right-hand column - page 3678, right-hand column * * page 3681 - page 3682 * * figures 4,5 * ----- | 1-31 | |
| X | ZHANG LIXIN ET AL: "Library on a slide for bacterial comparative genomics." BMC MICROBIOLOGY [ELECTRONIC RESOURCE]. ENGLAND 22 MAR 2004, vol. 4, no. 1, 22 March 2004 (2004-03-22), page 12, XP002286373 ISSN: 1471-2180 * abstract * * page 3 * * page 5 * ----- -/-- | 1-31 | TECHNICAL FIELDS SEARCHED (Int.Cl.7) C12Q |

The present search report has been drawn up for all claims

| Place of search | Date of completion of the search | Examiner |
|---|---|---|
| Munich | 3 August 2004 | Madlener, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

**European Patent Office**

**EUROPEAN SEARCH REPORT**

Application Number

EP 04 07 6309

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | YE R W ET AL: "Applications of DNA microarrays in microbial systems." JOURNAL OF MICROBIOLOGICAL METHODS. NETHERLANDS DEC 2001, vol. 47, no. 3, December 2001 (2001-12), pages 257-272, XP002286374 ISSN: 0167-7012 * abstract * * page 259 - page 264 * * page 265 - page 270 * * figures 1,2 * * tables 1,2 * ----- | 1-31 | |
| X | COSTA DE OLIVEIRA REGINA ET AL: "Competitive hybridization on spotted microarrays as a tool to conduct comparative genomic analyses of Xylella fastidiosa strains." FEMS MICROBIOLOGY LETTERS. NETHERLANDS 29 OCT 2002, vol. 216, no. 1, 29 October 2002 (2002-10-29), pages 15-21, XP002286375 ISSN: 0378-1097 * abstract * * page 15, last paragraph - page 16 * * page 20 - page 21 * ----- -/-- | 1-31 | **TECHNICAL FIELDS SEARCHED (Int.Cl.7)** |

The present search report has been drawn up for all claims

| Place of search | Date of completion of the search | Examiner |
|---|---|---|
| Munich | 3 August 2004 | Madlener, M |

EPO FORM 1503 03.82 (P04C01)

**European Patent Office**

## EUROPEAN SEARCH REPORT

Application Number

EP 04 07 6309

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | BEKAL SADJIA ET AL: "Rapid identification of Escherichia coli pathotypes by virulence gene detection with DNA microarrays." JOURNAL OF CLINICAL MICROBIOLOGY. UNITED STATES MAY 2003, vol. 41, no. 5, May 2003 (2003-05), pages 2113-2125, XP002286376 ISSN: 0095-1137 * abstract * * page 2114 * ----- | 1-31 | |
| X | MURRAY A E ET AL: "DNA/DNA hybridization to microarrays reveals gene-specific differences between closely related microbial genomes." PROCEEDINGS OF THE NATIONAL ACADEMY OF SCIENCES OF THE UNITED STATES OF AMERICA. UNITED STATES 14 AUG 2001, vol. 98, no. 17, 14 August 2001 (2001-08-14), pages 9853-9858, XP002286377 ISSN: 0027-8424 * abstract * * page 9854, right-hand column - page 9857, right-hand column * * figure 2 * * table 1 * ----- -/-- | 1-31 | TECHNICAL FIELDS SEARCHED (Int.Cl.7) |

The present search report has been drawn up for all claims

| Place of search | Date of completion of the search | Examiner |
|---|---|---|
| Munich | 3 August 2004 | Madlener, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

**European Patent Office**

**EUROPEAN SEARCH REPORT**

Application Number

EP 04 07 6309

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WU L ET AL: "Community Genome Arrays: An approach for detection and quantification of selected bacterial taxa in environmental samples" ABSTRACTS OF THE GENERAL MEETING OF THE AMERICAN SOCIETY FOR, vol. 102, 2002, page 387, XP009016870 102nd General Meeting of the American Society for Microbiology;Salt Lake City, UT, USA; May 19-23, 2002, 2002 ISSN: 1060-2011 * abstract * | 1-31 | |
| X | RAMISSE VINCENT ET AL: "DNA-DNA hybridization study of Burkholderia species using genomic DNA macro-array analysis coupled to reverse genome probing." INTERNATIONAL JOURNAL OF SYSTEMATIC AND EVOLUTIONARY MICROBIOLOGY, vol. 53, no. 3, May 2003 (2003-05), pages 739-746, XP009032338 ISSN: 1466-5026 (ISSN print) * abstract * * page 740, left-hand column - page 742 * * page 744, right-hand column, paragraph 2 * * tables 1,2 * | 1-31 | TECHNICAL FIELDS SEARCHED (Int.Cl.7) |

-/--

The present search report has been drawn up for all claims

| Place of search | Date of completion of the search | Examiner |
|---|---|---|
| Munich | 3 August 2004 | Madlener, M |

EPO FORM 1503 03.82 (P04C01)

## EUROPEAN SEARCH REPORT

**European Patent Office**

Application Number

EP 04 07 6309

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | XUE YONGTAO ET AL: "A DNA microarray for fission yeast: Minimal changes in global gene expression after temperature shift." YEAST, vol. 21, no. 1, 15 January 2004 (2004-01-15), pages 25-39, XP009034686 ISSN: 0749-503X (ISSN print) * abstract * * page 29 * | 1-31 | |
| X | WATANABE TAKAHITO ET AL: "A new approach to species determination for yeast strains: DNA microarray-based comparative genomic hybridization using a yeast DNA microarray with 6000 genes." YEAST (CHICHESTER, ENGLAND) ENGLAND MAR 2004, vol. 21, no. 4, March 2004 (2004-03), pages 351-365, XP009033471 ISSN: 0749-503X * abstract * | 1-31 | |
| A | AGGARWAL R K ET AL: "Two new genomes in the Oryza complex identified on the basis of molecular divergence analysis using total genomic DNA hybridization." MOLECULAR & GENERAL GENETICS: MGG. GERMANY 18 MAR 1997, vol. 254, no. 1, 18 March 1997 (1997-03-18), pages 1-12, XP002286380 ISSN: 0026-8925 * the whole document * | 1-31 | TECHNICAL FIELDS SEARCHED (Int.Cl.7) |

The present search report has been drawn up for all claims

| Place of search | Date of completion of the search | Examiner |
|---|---|---|
| Munich | 3 August 2004 | Madlener, M |

**EP 1 591 535 A1**

## ANNEX TO THE EUROPEAN SEARCH REPORT
## ON EUROPEAN PATENT APPLICATION NO.

EP 04 07 6309

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2004

| Patent document cited in search report | Publication date | Patent family member(s) | Publication date |
|---|---|---|---|
| US 2002187490 A1 | 12-12-2002 | WO 02101094 A1 | 19-12-2002 |

EPO FORM P0459